# EUROPEAN PATENT SPECIFICATION

(11) **EP 2 714 113 B1**
(45) Date of publication and mention of the grant of the patent: **15.04.2015**
(21) Application number: 12726225.1
(22) Date of filing: 16.05.2012
(51) Int. Cl.: A61L 27/60, C12N 5/00, A61L 27/38

(54) **HYDROGEL COMPOSITION AND USES THEREOF**
HYDROGELDZUSAMMENSETZUNG UND IHRE VERWENDUNG
COMPOSITION D'HYDROGEL ET SES UTILISATIONS

(30) Priority: 23.05.2011 IT TO20110450
(43) Date of publication of application: 09.04.2014
(73) Proprietor: Epinova Biotech S.r.l., 28100 Novara (IT)
(72) Inventor: RENÒ, Filippo, I-28100 Novara (IT); CANNAS, Mario, I-10138 Torino (IT)
(74) Representative: Freyria Fava, Cristina
(86) International application number: PCT/IB2012/052457
(87) International publication number: WO 2012/160484

(56) References cited:
- US-A1- 2011 052 693
- RENÒ F ET AL: "Functionalization of a poly(D,L)lactic acid surface with galactose to improve human keratinocyte behavior for artificial epidermis.", BIOTECHNOLOGY AND BIOENGINEERING 1 MAY 2008 LNKD- PUBMED:18080343, vol. 100, no. 1, 1 May 2008 (2008-05-01), pages 195-202, XP002663505, ISSN: 1097-0290
- PLACE ELSIE S ET AL: "Complexity in biomaterials for tissue engineering.", NATURE MATERIALS JUN 2009 LNKD- PUBMED:19458646, vol. 8, no. 6, June 2009 (2009-06), pages 457-470, XP002663506, ISSN: 1476-1122
- DRAPER BRADLEY K ET AL: "Epiregulin is more potent than EGF or TGFalpha in promoting in vitro wound closure due to enhanced ERK/MAPK activation.", JOURNAL OF CELLULAR BIOCHEMISTRY 15 AUG 2003 LNKD- PUBMED:12898511, vol. 89, no. 6, 15 August 2003 (2003-08-15), pages 1126-1137, XP002663507, ISSN: 0730-2312
- HASHIMOTO K: "Regulation of keratinocyte function by growth factors.", JOURNAL OF DERMATOLOGICAL SCIENCE DEC 2000 LNKD- PUBMED:11137396, vol. 24 Suppl 1, December 2000 (2000-12), pages S46-S50, XP002663508, ISSN: 0923-1811

### Sequence Listing

<110> University degli Studi del Piemonte Orientale "Amedeo Avogadro"
<120> Hydrogel composition and uses thereof
<130> BWO14322-CF
<160> 12
<170> PatentIn version 3.5
<210> 1
   <211> 20
   <212> DNA
   <213> artificial
<220>
   <223> K5 - forward primer
<400> 1
   caccaagact gtgaggcaga 20
<210> 2
   <211> 20
   <212> DNA
   <213> artificial
<220>
   <223> K5 - reverse primer
<400> 2
   catccatcag tgcatcaacc 20
<210> 3
   <211> 20
   <212> DNA
   <213> artificial
<220>
   <223> K10 - forward primer
<400> 3
   gaaccacgag gaggaaatga 20
<210> 4
   <211> 20
   <212> DNA
   <213> artificial
<220>
   <223> K10 - reverse primer
<400> 4
   aactgttctt ccagagcgga 20
<210> 5
   <211> 20
   <212> DNA
   <213> artificial
<220>
   <223> MMP-28 - forward primer
<400> 5
   agagcgtttc agtgggtgtc 20
<210> 6
   <211> 20
   <212> DNA
   <213> artificial
<220>
   <223> MMP-28- reverse primer
<400> 6
   aaagcgtttc ttacgcctca 20
<210> 7
   <211> 20
   <212> DNA
   <213> artificial
<220>
   <223> Filaggrin - forward primer
<400> 7
   tgatgcagtc tccctctgtg 20
<210> 8
   <211> 20
   <212> DNA
   <213> artificial
<220>
   <223> Filaggrin - reverse primer
<400> 8
   tgtttctctt gggctcttgg 20
<210> 9
   <211> 20
   <212> DNA
   <213> artificial
<220>
   <223> TG-1 - forward primer
<400> 9
   acgttactgg gagcagcagt 20
<210> 10
   <211> 20
   <212> DNA
   <213> artificial
<220>
   <223> TG-1 - reverse primer
<400> 10
   tgccataggg atggtctctc 20
<210> 11
   <211> 23
   <212> DNA
   <213> artificial
<220>
   <223> beta-actin - forward primer
<400> 11
   acactgtgcc catctacgag ggg 23
<210> 12
   <211> 29
   <212> DNA
   <213> artificial
<220>
   <223> beta-actin - reverse primer
<400> 12
   atgatggagt tgaaggtagt ttcgtggat 29

## Description

### Field of the invention

This disclosure concerns a hydrogel for tissue engineering applications, in particular for creation of artificial skin.

### Background of the invention

Skin is the largest organ of the body in vertebrates and represents a physiological barrier against exogenous substances, pathogens and mechanical stress. Defects in this barrier integrity cause water and protein loss, and allow bacteria to invade the underlying tissue: in fact, after major trauma, such as burns, the absence of keratinocytes and the biological barrier they form, causes different clinical problems such as infections and wound hypertrophy.

Tissue engineering has provided many alternative therapies, including cultured allogenic and autologous engineered skin, to conventional wound closure in patients suffering from major trauma where donors sites are limited and require multiple harvesting procedures.

Studies on the development of artificial epidermis started from the observation that keratinocytes can be grown in culture to produce thin epithelial sheet grafts using different experimental procedures.

A more useful technical approach to the development of artificial epidermis concerns the use of suitable biocompatible and biodegradable scaffolds, such as collagen, hyaluronic acid and biopolymers capable to favour keratinocytes growth onto their surface. Keratinocytes growth is, nevertheless, unsatisfactory on these scaffolds because keratinocytes are unable to generate blood vessels, have reduced mechanical capabilities and are unable to create differentiated structures, such as sebaceous glands, melanocites and Langherans cells; thus such keratinocytes cannot be used as natural skin substitutes.

### Summary of the invention

Taking into account these premises, the need is therefore felt for improved solutions enabling tissue engineering, specifically skin engineering.

The object of this disclosure is providing such improved solutions.

According to the invention, the above object is achieved thanks to the subject matter recalled specifically in the ensuing claims, which are understood as forming an integral part of this disclosure.

An embodiment of the present disclosure provides a new hydrogel composition suitable for human keratinocytes growth, wherein keratinocytes are able to adhere, proliferate and stratify.

More specifically, the present disclosure concerns an hydrogel composed of gelatin, poly-glutamic acid supplemented with epiregulin, a member of the epithelial growth factors family, for obtaining human keratinocytes adhesion, proliferation and stratification.

A further embodiment of the present disclosure provides for use of an hydrogel composed of gelatin, poly-glutamic acid and epiregulin for the preparation of a hydrogel scaffold suitable for cultivation of cells, preferably keratinocytes, more preferably human keratinocytes.

A still further embodiment of the present disclosure provides for a process for cultivating keratinocytes, preferably human keratinocytes, comprising use of a hydrogel scaffold, wherein the hydrogel scaffold is realized using an hydrogel composed of gelatin, poly-glutamic acid and epiregulin.

### Brief description of the drawings

The invention will now be described, by way of example only, with reference to the enclosed figures of drawing, wherein:
- **Figure 1****:** HaCaT adhesion onto control and epiregulin-enriched hydrogel at 6 hours post-seeding. Magnification = 10X
- **Figure 2****:** HaCaT morphology onto HG and epiregulin-enriched HG at 7 days post-adhesion. Magnification = 10X.
- **Figure 3****:** Quantification of HaCaT proliferation at 3 (white bars) and 7 (black bars) days postadhesion by colorimetric assay (CellTiter-Blue). ** p<0.001, * p<0.05 compared to control samples.
- **Figure 4****: A)** Representative fluorescence image of a transversal section (25 µm) of HaCaT epithelium at 7 days post-adhesion onto control and 50 ng/ml epiregulin-enriched hydrogel, respectively. Magnification = 20X. **B)** Quantification of the epithelial thickness. * p<0.05.
- **Figure 5****: A)** Reprentative TBE-agarose gel for RT-PCR amplified K5, K10 and β-actin transcripts in HaCaT cells 7 day post-adhesion onto control and epiregulin-enriched hydrogel. **B)** Densitometric quantification of K5 and K10 expression. β-action was used as housekeeping gene for data normalization. Cells were grown onto hydrogels enriched with different concentration of epiregulin: no epiregulin (control, white bars), 5 ng/ml (grey bars), 10 ng/ml (light grey bars) and 50 ng/ml (black bars).
- **Figure 6****: A)** Representative TBE-agarose gel for RT-PCR amplified MMP-28, TG-1, Filaggrin and β-actin transcripts in HaCaT cells 7 day post-adhesion onto control and epiregulin-enriched hydrogel. **B)** Densitometric quantification of MMP-28, TG-1 and Filaggrin expression. β-action was used as housekeeping gene for data normalization. Cells were grown onto hydrogels enriched with different concentration of epiregulin: no epiregulin (control, white bars), 5 ng/ml (striped bars), 10 ng/ml (grey bars) and 50 ng/ml (black bars).
- **Figure 7****: A)** Gelatin zymography highlighting MMP-2 and MMP-9 activity in conditioned cell culture medium from HaCaT grown onto control or epiregulin-enriched hydrogels for 7 days. **B)** Densitometric quantification of MMP-2 and MMP-9 activity at 7 days post-adhesion. White bars represent MMP-2, black bars represent MMP-9 activity, respectively.

### Detailed description of the invention

In the following description, numerous specific details are given to provide a thorough understanding of embodiments. The embodiments can be practiced without one or more of the specific details, or with other methods, components, materials, etc. In other instances, well-known structures, materials, or operations are not shown or described in detail to avoid obscuring aspects of the embodiments.

Reference throughout this specification to "one embodiment" or "an embodiment" means that a particular feature, structure, or characteristic described in connection with the embodiment is included in at least one embodiment. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

The headings provided herein are for convenience only and do not interpret the scope or meaning of the embodiments.

Tissue engineering provides solutions for tissue loss, or restoration of tissue or organ function with constructs that contain specific populations of living cells.

Since tissue loss at the skin level is a common occurrence due to a plethora of events such as lacerations, cutaneous diseases, neoplasia, infection, burns and other trauma, adequate cutaneous constructs that could act as effective skin replacements, capable of mimicking native skin, are highly desirable.

Many natural biopolymers, such as glycosaminoglycans, collagen, fibronectin and hyaluronan, are currently used as potential scaffolds for keratinocyte adhesion and proliferation, but the lack of blood vessels, their reduced mechanical capabilities and the absence of differentiated structures, such as sebaceous glands, melanocites and Langherans cells, impair their use as natural skin substitutes.

Recently, among the "natural scaffolds" for tissue engineering applications, ionic hydrogels have been extensively studied.

Ionic hydrogels are polymeric materials with a porous tridimensional structure, made by crosslinking different natural and synthetic compounds, obtaining a highly hydrated structure, highly biocompatible and biodegradable. Moreover these scaffolds display a good swelling so they are interesting candidates for many biological applications.

An embodiment of the present disclosure concerns a gelatin-based hydrogel loaded with a specific epithelial growth factor, epiregulin (Epi), to enhance and/or to speed the formation of a functional epithelium for use in the treatment of e.g. burns or chronic wounds.

The aim of the present disclosure is the use of this anionic hydrogel formed by crosslinking gelatin with poly-L-glutamic acid as a novel substrate for human keratinocyte growth.

The data provided herein indicate that the anionic hydrogel used as scaffold for keratinocyte growth allowed HaCaT cells adhesion and growth and that epiregulin presence significantly increased keratinocyte growth and stratification after one week.

Epiregulin stimulates human keratinocyte proliferation under both subconfluent and confluent culture conditions in the absence of exogenous EGF family growth factors.

Epidermal regeneration is a complex process in which residual epithelial cells proliferate and migrate in an integrated fashion to regenerate intact epidermis.

The increase in cell proliferation observed onto Epi-loaded hydrogel (HG) was confirmed by the increased expression of K5, a cytokeratine expressed by basal proliferating keratinocytes and by the simultaneous decrease in K10 expression, a marker of keratinocyte differentiation.

Furthermore cells grown onto Epi-loaded HG showed a lowering in the expression of filaggrin and TG-1, other markers of keratinocyte differentiation.

Filaggrin is an abundant and important protein expressed in differentiating keratinocytes that functions to aggregate keratin interfilaments into aligned macrofibrils in the stratum corneum of the epidermis, while TG-1 is a key enzyme mainly expressed in skin granular layer and it catalyzes the crosslinking of several proteins for the formation of the cellular envelope.

Therefore Epi seems to lead keratinocyte toward a proliferative and less differentiated phenotype.

Another important aspect in wound healing is the ability of keratinocytes to migrate to reconstruct the epithelium continuity.

The hydrogel loaded with epiregulin according to the instant invention allowed the growth of a stratified human epithelium, that likely withstand increased stress and trauma thus allowing its use in the process of epithelialization of skin damages.

Key enzymes involved in wound healing are matrix metalloproteinases (MMPs), in particular gelatinase A (MMP-2) and gelatinase B (MMP-9) while another MMP, epilysin (MMP-28), resulted involved in wound healing, even if its role is still unclear.

During wound healing a number of migratory and remodelling events that require the action of MMPs and their natural inhibitors, the tissue inhibitors of metalloproteinases, occur.

MMP-28, also known as Epilysin, is a proteolytic enzyme belonging to the matrix metalloproteinases (MMPs) family, selectively produced by keratinocytes which expression is upregulated during wound healing and hypertrophic scaring. Epilysin expression is induced during wound healing, in migrating keratinocytes and in basal stationary keratinocytes far behind the wound edge, implying a role for epilysin both in normal tissue homeostasis and in response to injury.

Human keratinocytes grown onto Epi-enriched HG showed a reduced MMP-2 production and an increased MMP-9 expression indicating that the presence of epiregulin induced a more proliferating epithelium while reducing the potential migratory ability of keratinocytes. Moreover the presence of epiregulin slightly increased the expression of MMP-28, normally expressed in basal proliferating keratinocytes.

The hydrogel composition object of the instant application can be employed in the creation of artificial skin for treatment of skin diseases or damages, by seeding the hydrogel scaffold, realized using the herein described hydrogel composition, with human keratinocytes obtained by biopsic samples of the patient in need of such a treatment.

The hydrogel composition object of the instant application can also be employed in the creation of artificial skin for laboratory tests, mainly for evaluating the toxic effects exerted by substances under study.

### Materials and Methods

### Synthesis of gelatin hydrogels (HG)

A 10% w/v gelatin solution was prepared by adding 100 mg of gelatin type B (Gelatin type B from bovine skin, Sigma (∼ 300 bloom; IEP = 9,0)) to 1 ml phosphate-buffered saline solution (PBS; pH=7.4). The mixture was heated to 40°C for 10 minutes to allow complete gelatin dissolution.

Following gelatin dissolution 10 mg of PLG (PLG, Sigma (MW = 50000-100000)) were added. EDC (1-[3-(dimethylamino) propyl]-3-ethylcarbodiimide, Sigma Aldrich) and NHS (Nhydroxysuccinimide, Sigma Aldrich) solutions were added in 4:1 molar ratio in PBS. After crosslinking reaction, hydrogels were washed in PBS to remove the unreacted by-products and the urea derivative of EDC. In order to obtain HG enriched with epiregulin (Epi) (Recombinant human Epiregulin, R&D Systems, cat.num. 1195-EP), various amounts of growth factor (5-50 ng/ml in PBS) were added during crosslinking process. Hydrogels were produced in sheets (surface = 10 cm², width ∼ 1 mm) and then cut in 1 cm² square samples.

### Human keratinocyte adhesion and proliferation

Spontaneously immortalized keratinocytes (HaCaT), isolated from human adult skin [P. Boukamp, R.T. Petrussevska, D. Breitkreutz, J. Hornung, A. Markham, N.E. Fusenig. J. Cell Biol. 106, 761 (1988)] were a kind gift of Dr. M. De Andrea from the Microbiology Laboratory, University of Eastern Piedmont ''A. Avogadro''.

Cells were grown in culture flask (75 cm²) in DMEM medium (Euroclone, Milan, Italy) supplemented with 10% heat inactivated foetal bovine serum (FBS) (Euroclone), penicillin (100 U/mL), streptomycin (100 mg/mL) and L-glutamine (2 mM) (Euroclone) in a humidified atmosphere containing 5% CO₂ at 37°C. A volume of 200 µL of HaCaT cells suspension (50x10⁴ cell/mL) in serum-free DMEM was added to HG samples (sample surface area = 1 cm²).

After incubation, unattached cells were removed by washing with PBS and 1 ml of fresh medium was added (DMEM with 10% FBS). Cell adhesion was assessed by counting cells in 10 fields at 10X magnification (field area = 3 mm²) and expressed as adherent cells/cm².

After 3 and 7 days cell proliferation was quantified both by morphological observation taking 10 random microscope images with a 10X magnification across the sample surface using a Leica DM2500 microscope and using a fluorimetric method based on the quantification of resazurin reduction by viable cells (CellTiter-Blue, Promega). Briefly, 25 µl of dye were dissolved in 0.5 ml complete cell growth medium and after 4 hours the optical density was measured at 620 nm.

Cell proliferation was expressed as optical density (O.D.) variation ± standard deviation (S.D.). At day 7 hydrogel samples were fixed for 15 min at room temperature using a solution of formaldehyde (3.7%) and sucrose (3%) in PBS (pH 7.4). After fixation samples were embedded in cryoblock medium and 25 µm sections were cut using Leica CM 1850 Cryostat. Sections were stained with 2 µg/ml of Hoechst 33342 solution in order to stain cell nuclea and then observed under UV light using fluorescence microscope Leica DM 2500.

### Reverse transcriptase polymerase chain reaction

Total cellular RNA was extracted using Quiagen RNeasy MicroKit (Quiagen, Germany) following manufacturer's protocol. Complementary DNA (cDNA) were prepared from total RNA using 0.5 µg random primers (Promega, catalog number: C118B) in a final volume of 20 µl. The reaction mixture was heated at 70°C for 5 minute and then 25 mM MgCl2, 5X reaction buffer (Promega), 10 mM dNTPs mix (Promega), 20 U ribonuclease inhibitor (Promega) and RT enzyme (Promega) were added. The reaction was performed at 42 °C for 1h. The resulting cDNA was used for the polymerase chain reaction (PCR). Synthetic oligonucleotide primers specific for human Epilysin (MMP-28), Filaggrin (Fil), Transglutaminase-1 (TG-1), cytokeratin 5 (K5), cytokeratin 10 (K10) and β-action were designed using the primer-blast tool software from NCBI. Forward and reverse primer sequences and the expected size of PCR products are indicated in Table 1. Reaction products were separated onto 1.8% TBE-agarose gel and stained with ethidium bromide. DNA molecular weight marker (Sigma Aldrich, catalog number:P1473 PCR 100 bp Low Ladder) was used as a size marker.

**Table 1.**

| **Gene** | **Primers** | **SEQ ID No. :** | **Product size** |
|---|---|---|---|
| **K5** | For. 5'-CAC CAA GAC TGT GAG GCA GA-3' | 1 | 274 bp |
| | Rev. 5'-CAT CCA TCA GTG CAT CAA CC -3' | 2 | |
| **K10** | For. 5'-GAA CCA CGA GGA GGA AAT GA-3' | 3 | 398 bp |
| | Rev. 5'-AAC TGT TCT TCC AGA GCG GA-3' | 4 | |
| **MMP-28** | For. 5'-AGA GCG TTT CAG TGG GTG TC-3' | 5 | 180 bp |
| | Rev. 5'-AAA GCG TTT CTT ACG CCT CA-3' | 6 | |
| **Filaggrin** | For. 5'-TGA TGC AGT CTC CCT CTG TG-3' | 7 | 338 bp |
| | Rev. 5'-TGT TTC TCT TGG GCT CTT GG-3' | 8 | |
| **TG-1** | For. 5'-ACG TTA CTG GGA GCA GCA GT-3' | 9 | 351 bp |
| | Rev. 5'-TGC CAT AGG GAT GGT CTC TC-3' | 10 | |
| **β**-**actin** | For. 5'-ACA CTG TGC CCA TCT ACG AGG GG-3' | 11 | 360 bp |
| | Rev. 5'-ATG ATG GAG TTG AAG GTA GTT TCG TGG AT-3' | 12 | |

### Gelatin zymography

To detect MMP-2 and MMP-9 activity, conditioned media from HaCaT cells grown onto control and epiregulin enriched HG were separated by electrophoresis on SDS-polyacrylamide gels containing 0.2% gelatin. Samples were loaded onto zymograms without denaturation. After running, gels were washed at room temperature for 2 h in 2.5% Triton X-100 solution and incubated overnight at 37°C in 0.5 M Tris-HCl, 0.2 M NaCl, 5 mM CaCl₂, 1 mM ZnCl₂ buffer.

Gels were then fixed in MeOH/Acetic Acid (50:10) solution and stained in 0.5% Coomassie Blue in MeOH/Acetic Acid (40:10) solution. Images of stained gels were acquired after appropriate destaining. Gelatinolytic activity was detected as white bands on a dark blue background and quantified by densitometric analysis using ImageJ software.

### Statistical analysis

The statistical analysis of data was performed using the Graph Pad Prism 2.01 software for Windows using the Anova test followed by Dunnett's post-hoc test. Probability values of p<0.05 were considered statistically significant. Experiments were done in triplicate and data were expressed as mean values ± standard deviation (S.D.)

### Results

### Cell adhesion and proliferation

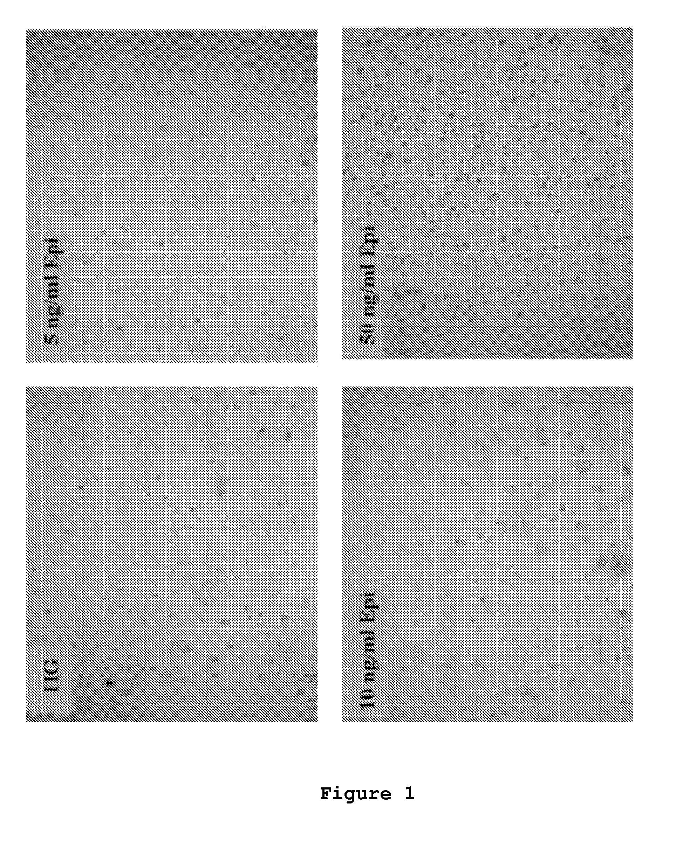

As shown in figure 1, Epiregulin (Epi) incorporation into HG did not significantly modify HaCaT adhesion after 6 hours. In fact the average number of cells adhered onto HGs were 56700 ± 5800 cells/cm² in control samples, while 46027 ± 6200, 50348 ± 5900 and 45111 ± 7100 cells/cm² for 5, 10, 50 ng/ml Epi-enriched HG, respectively.

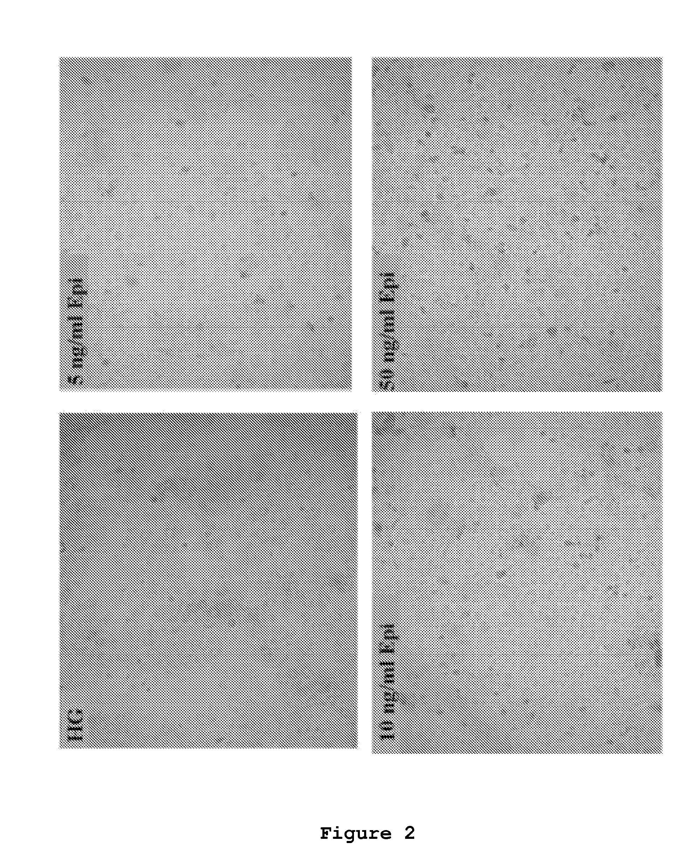

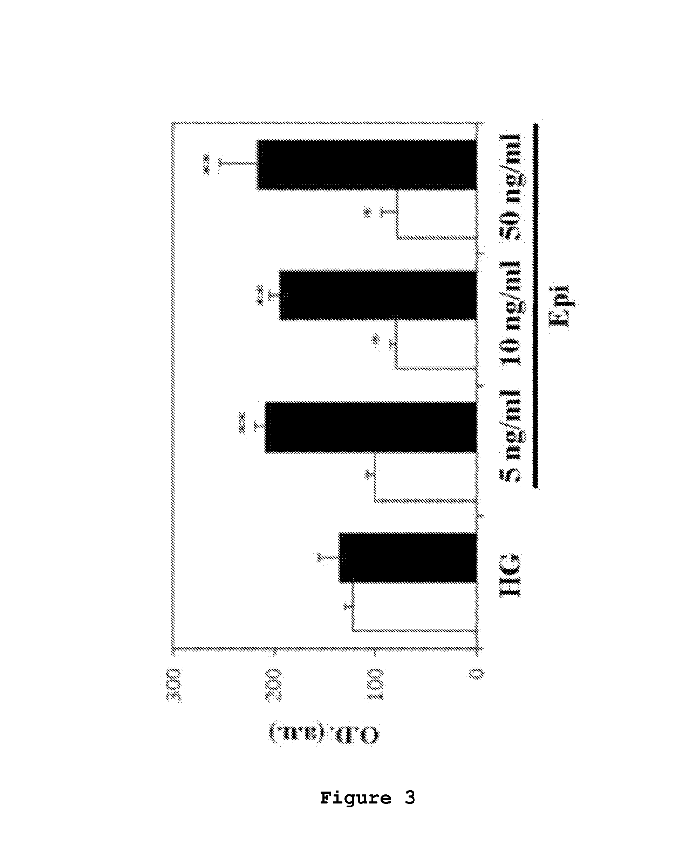

At 3 days post-adhesion, a slight decrease in HaCaT proliferation occurred compared to cells seeded onto hydrogels containing the highest Epi concentrations (p<0.05 compared to control samples), but this effect was completely reverted at 7 days post-adhesion. In fact, as shown both in figure 2 and figure 3, proliferation of HaCaT cells seeded onto Epi-enriched HG was higher than that observed for HaCaT cells seeded onto control HG (p<0.001). It is noteworthy that the maximum proliferative effect was already reached at the lowest Epi concentration.

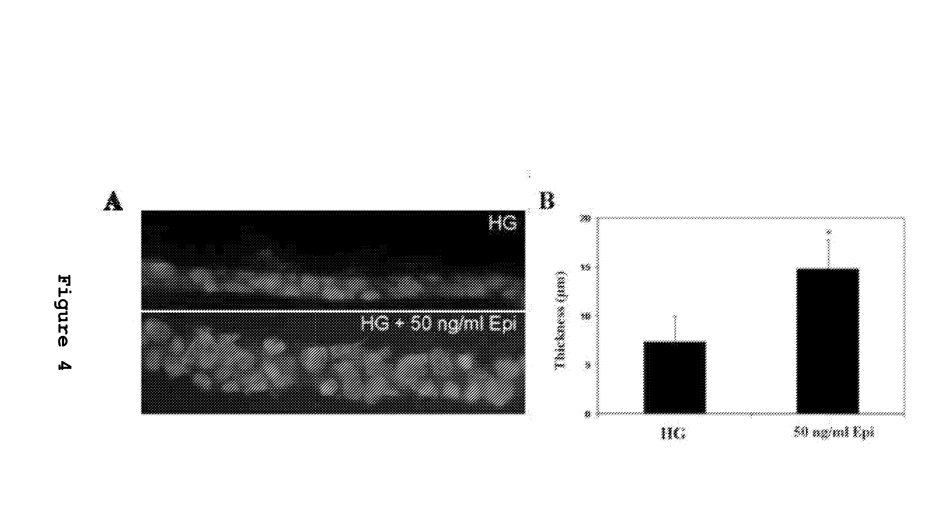

At 7 days post-adhesion HaCaT cells grown both onto control and Epi-enriched HGs formed a continuous epithelium. In order to evaluate its structure samples were fixed, included in cryoblock medium and 25 µm sections were cut using a cryostat. Slides were then stained with Hoechst 33342 solution in order to stain keratinocytes nuclea as shown in figure 4A.

Epithelium grown onto all the Epi-enriched HGs was thicker than that grown onto control HG. In fact morphometrical analysis performed onto 50 ng/ml Epi-enriched HG sections (figure 4B) highlighted that epithelium grown onto this HG was 14.9 ± 2.9 µm thick, while the one grown onto control HG was only 7.3 ± 2.8 µm thick (p<0.05). Similar results were obtained for both 5 and 10 ng/ml Epi-enriched HGs.

### Proliferation and differentiation markers expression in cells grown onto Epi-enriched HGs

Total RNA was extracted from keratinocytes grown for 7 days onto control and Epi-enriched (5, 10, 50 ng/ml) HGs. RNA was reverse-transcribed and amplifyed by PCR to assess cytokeratin 5 (K5), cytokeratin 10 (K10), filaggrin, transglutaminase-1 (TG-1) and epilysin (MMP-28) expression. β-action was used as housekeeping gene.

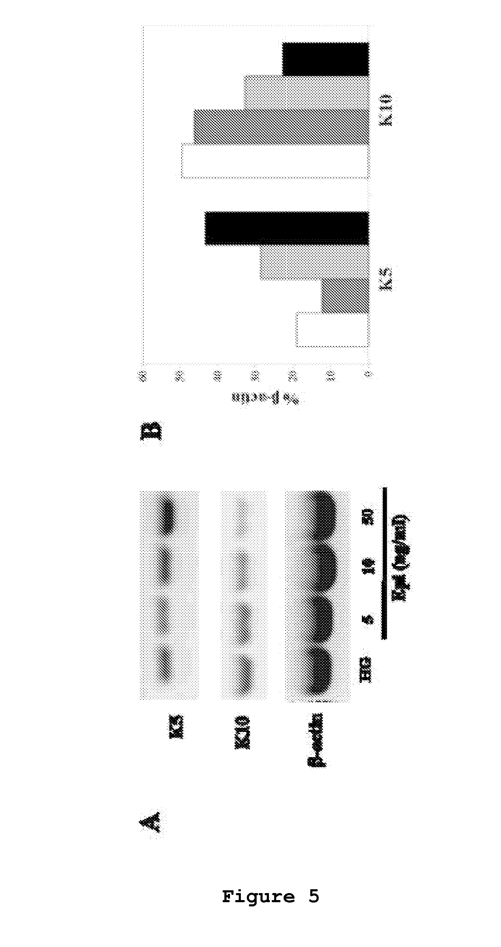

As shown in figures 5A and B, Epi addition to HG deeply modified cytokeratin expression in HaCaT cells. In particular K5 expression, which usually correlate with proliferanting keratinocytes, was doubled at 7 days by 50 ng/ml Epi as observed by densitometric analysis, while the differentiation marker K10 expression was halved.

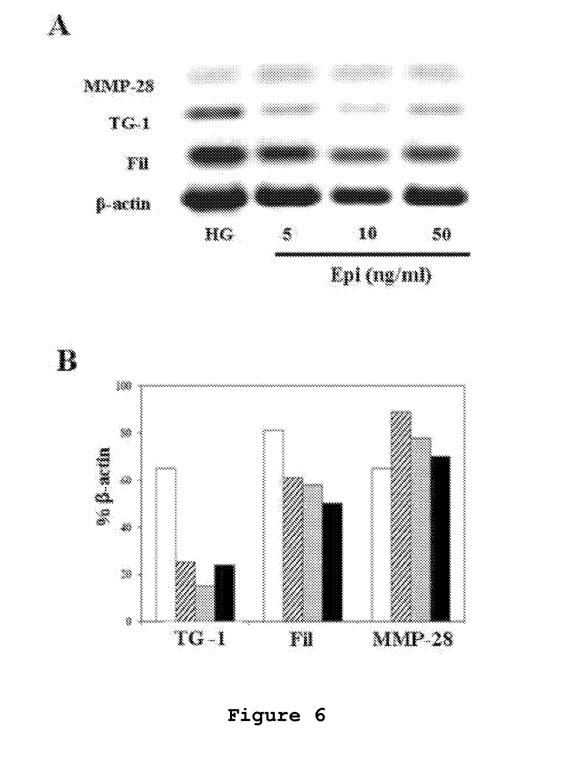

According to this anti-differentiation effect, also the expression of other two differentiation markers (filaggrin and TG-1) appeared reduced in a dose dependent manner (figure 6A, B), while the expression of epilysin (MMP-28), a matrix metalloproteinase expressed by basal and suprabasal keratinocytes, was slightly increased in HaCaT cells grown onto Epi-enriched HGs.

### MMP-2 and MMP-9 activity

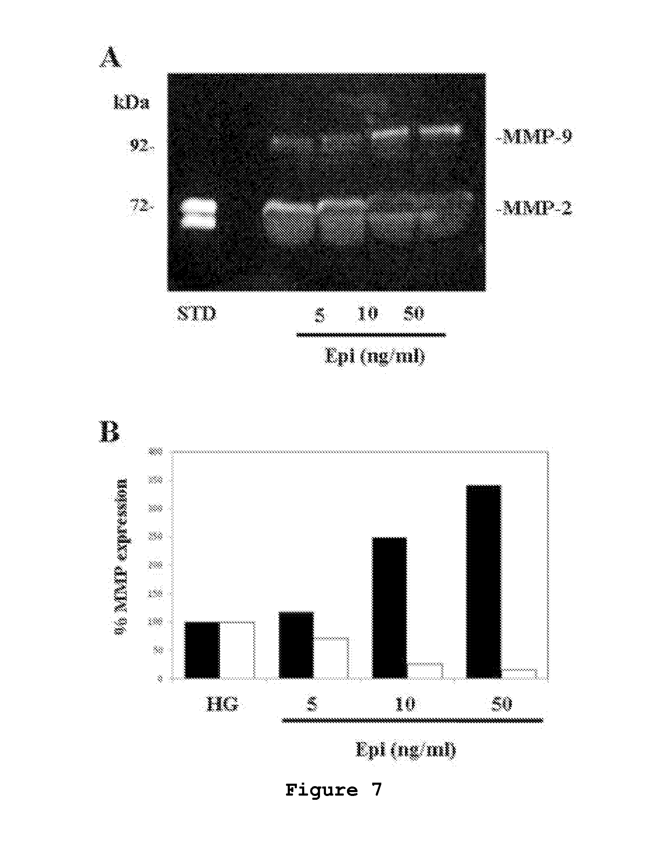

The presence and activity of MMP-2 and MMP-9, two Zn²⁺ dependent preoteases known to be involved in cell migration, was assessed, by means of gelatine zymography, in conditioned cell culture medium from cells grown both onto control and Epi-enriched HGs for 7 days, as shown in figure 7A.

HaCaT cells grown onto Epi-enriched HG produced a significantly higher amount of MMP-9 starting from 10 ng/ml Epi concentration, while MMP-2 expression was reduced in a similar manner. In particular, densitometric analysis indicated that, in cells grown onto 50 ng/ml Epi-enriched HG, both an increase of more than 350% of MMP-9 and a 80% decrease in MMP-2 production occurred compared to control sample (figure 7B). Those data indicated that the presence of Epi in HG fostered a proliferative behavior in HaCaT cells.

Naturally, while the principle of the invention remains the same, the details of construction and the embodiments may widely vary with respect to what has 5 been described and illustrated purely by way of example, without departing from the scope of the present invention.

## Claims

1. Hydrogel composition, said composition comprising gelatin, poly-glutamic acid and epiregulin.

2. Hydrogel composition according to claim 1, wherein the gelatin is selected between gelatin A and gelatin B.

3. Hydrogel composition according to claim 1 or claim 2, wherein poly-glutamic acid is poly-L-glutamic acid.

4. Hydrogel composition according to any one of the preceding claims, wherein gelatin is present in an amount between 50 and 150 mg/ml, preferably between 90 and 110 mg/ml.

5. Hydrogel composition according to any one of the preceding claims, wherein poly-glutamic acid is present in an amount between 5 and 15 mg/ml, preferably between 9 and 11 mg/ml.

6. Hydrogel composition according to any one of the preceding claims, wherein epiregulin is present in a concentration of 1 to 100 ng/ml, preferably 5 to 50 ng/ml.

7. Hydrogel composition according to any one of the preceding claims, wherein said composition further comprises at least one of 1-[3-(dimethylamino)propyl]-3-ethylcarbodiimide (ECD) and N-hydroxysuccinimide (NHS).

8. Hydrogel composition according to any one of the preceding claims, wherein poly-glutamic acid has a molecular weight in the range of 50,000-100,000.

9. Use of a hydrogel composition according to any one of claims 1 to 8 for the preparation of a hydrogel scaffold suitable for cultivation of cells, preferably keratinocytes, more preferably human keratinocytes.

10. Process for cultivating keratinocytes, preferably human keratinocytes, the process comprising i) realizing a hydrogel scaffold using a hydrogel composition according to any one of claims 1 to 8 and ii) seeding keratinocytes on said scaffold.

11. Process according to claim 10, wherein the hydrogel scaffold is realized by mixing a gelatin solution with poly-glutamic acid and epiregulin and crosslinking the obtained mixture.

## Patentansprüche

1. Hydrogelzusammensetzung, wobei die Zusammensetzung Gelatine, Polyglutaminsäure und Epiregulin umfasst.

2. Hydrogelzusammensetzung nach Anspruch 1, wobei die Gelatine aus Gelatine A und Gelatine B ausgewählt ist.

3. Hydrogelzusammensetzung nach Anspruch 1 oder Anspruch 2, wobei die Polyglutaminsäure Poly-L-glutaminsäure ist.

4. Hydrogelzusammensetzung nach einem der vorangehenden Ansprüche, wobei die Gelatine in einer Menge zwischen 50 und 150 mg/ml, vorzugsweise zwischen 90 und 110 mg/ml vorhanden ist.

5. Hydrogelzusammensetzung nach einem der vorangehenden Ansprüche, wobei die Polyglutaminsäure in einer Menge zwischen 5 und 15 mg/ml, vorzugsweise zwischen 9 und 11 mg/ml vorhanden ist.

6. Hydrogelzusammensetzung nach einem der vorangehenden Ansprüche, wobei das Epiregulin in einer Konzentration von 1 bis 100 ng/ml, vorzugsweise 5 bis 50 ng/ml vorhanden ist.

7. Hydrogelzusammensetzung nach einem der vorangehenden Ansprüche, wobei die Zusammensetzung außerdem wenigstens eines von 1-[3-(Dimethylamino)propyl]-3-ethylcarbodiimid (ECD) und N-Hydroxysuccinimid (NHS) umfasst.

8. Hydrogelzusammensetzung nach einem der vorangehenden Ansprüche, wobei die Polyglutaminsäure ein Molekulargewicht im Bereich von 50000-100000 aufweist.

9. Verwendung einer Hydrogelzusammensetzung nach einem der Ansprüche 1 bis 8 für die Herstellung eines Hydrogelgerüsts, das sich für die Kultivierung von Zellen, vorzugsweise Keratinozyten, stärker bevorzugt menschlichen Keratinozyten eignet.

10. Verfahren zur Kultivierung von Keratinozyten, vorzugsweise menschlichen Keratinozyten, wobei das Verfahren i) das Verwirklichen eines Hydrogelgerüsts unter Verwendung einer Hydrogelzusammensetzung nach einem der Ansprüche 1 bis 8 und ii) das Einsäen von Keratinozyten auf das Gerüst umfasst.

11. Verfahren nach Anspruch 10, wobei das Hydrogelgerüst durch Vermischen einer Gelatinelösung mit Polyglutaminsäure und Epiregulin und Vernetzen der erhaltenen Mischung verwirklicht wird.

## Revendications

1. Composition d'hydrogel, ladite composition comprenant de la gélatine, de l'acide poly-glutamique et de l'épiréguline.

2. Composition d'hydrogel selon la revendication 1, dans laquelle la gélatine est choisie entre une gélatine A et une gélatine B.

3. Composition d'hydrogel selon la revendication 1 ou 2, dans laquelle l'acide poly-glutamique est l'acide poly-L-glutamique.

4. Composition d'hydrogel selon l'une quelconque des revendications précédentes, dans laquelle la gélatine est présente en une quantité comprise entre 50 et 150 mg/ml, de préférence entre 90 et 110 mg/ml.

5. Composition d'hydrogel selon l'une quelconque des revendications précédentes, dans laquelle l'acide poly-glutamique est présent en une quantité comprise entre 5 et 15 mg/ml, de préférence entre 9 et 11 mg/ml.

6. Composition d'hydrogel selon l'une quelconque des revendications précédentes, dans laquelle l'épiréguline est présente en une concentration de 1 à 100 ng/ml, de préférence de 5 à 50 ng/ml.

7. Composition d'hydrogel selon l'une quelconque des revendications précédentes, dans laquelle ladite composition comprend en outre au moins l'un parmi 1-[3-(diméthylamino)propyl]-3-éthylcarbodiimide (ECD) et N-hydroxysuccinimide (NHS).

8. Composition d'hydrogel selon l'une quelconque des revendications précédentes, dans laquelle l'acide poly-glutamique a un poids moléculaire se trouvant dans la plage allant de 50 000 à 100 000.

9. Utilisation d'une composition d'hydrogel selon l'une quelconque des revendications 1 à 8 pour la préparation d'une structure d'hydrogel appropriée pour la mise en culture de cellules, de préférence de kératinocytes, plus préférablement de kératinocytes humains.

10. Procédé de mise en culture de kératinocytes, de préférence de kératinocytes humains, le procédé comprenant
i) la réalisation d'une structure d'hydrogel en utilisant une composition d'hydrogel selon l'une quelconque des revendications 1 à 8 et
ii) l'ensemencement de kératinocytes sur ladite structure.

11. Procédé selon la revendication 10, dans lequel la structure d'hydrogel est réalisé par mélange d'une solution de gélatine avec de l'acide poly-glutamique et de l'épiréguline et la réticulation du mélange obtenu.
